(19) Europäisches Patentamt
European Patent Office
Office européen des brevets

(11) **EP 1 623 667 B1**

(12) **EUROPEAN PATENT SPECIFICATION**

(45) Date of publication and mention
of the grant of the patent:
**13.06.2012   Bulletin 2012/24**

(51) Int Cl.:
*A61B 5/024* (2006.01)       *A61B 5/11* (2006.01)
*A61B 5/0245* (2006.01)

(21) Application number: **05254872.4**

(22) Date of filing: **04.08.2005**

(54) **A non contact measurement technique for the monitoring of a physiological condition**

Technik zur kontaktlosen Überwachung eines physiologischen Zustands

Technique de mesure sans contact pour surveiller l'état physiologique

(84) Designated Contracting States:
**AT BE BG CH CY CZ DE DK EE ES FI FR GB GR HU IE IS IT LI LT LU LV MC NL PL PT RO SE SI SK TR**

(30) Priority: **06.08.2004   GB 0417590**

(43) Date of publication of application:
**08.02.2006   Bulletin 2006/06**

(73) Proprietors:
  • **Scalise, Lorenzo**
    **60100 Ancona (IT)**
  • **Grigioni, Mauro**
    **00151 Roma (IT)**
  • **Morbiducci, Umberto**
    **62100 Macerata (IT)**
  • **Tomasini, Enrico Primo**
    **63033 Monteprandone AP (IT)**

(72) Inventors:
  • **Scalise, Lorenzo**
    **60100 Ancona (IT)**
  • **Grigioni, Mauro**
    **00151 Roma (IT)**
  • **Morbiducci, Umberto**
    **62100 Macerata (IT)**
  • **Tomasini, Enrico Primo**
    **63033 Monteprandone AP (IT)**

(74) Representative: **Baldi, Claudio**
    **Viale Cavallotti, 13**
    **60035 Jesi (AN) (IT)**

(56) References cited:
    **US-A1- 2003 135 097**

  • **MEIGAS K ET AL: "Self-mixing in a diode laser as a method for cardiovascular diagnostics" JOURNAL OF BIOMEDICAL OPTICS SPIE USA, vol. 8, no. 1, January 2003 (2003-01), pages 152-160, XP002352578 ISSN: 1083-3668**
  • **SCALISE L: "Self-mixing feedback Doppler vibrometry" PROCEEDINGS OF THE SPIE - THE INTERNATIONAL SOCIETY FOR OPTICAL ENGINEERING SPIE-INT. SOC. OPT. ENG USA, vol. 4827, 2002, pages 374-384, XP002352579 ISSN: 0277-786X**
  • **CASTELLINI P ET AL: "Application of lasers for noncontact excitation and measurement of vibration" PROCEEDINGS OF THE SPIE - THE INTERNATIONAL SOCIETY FOR OPTICAL ENGINEERING SPIE-INT. SOC. OPT. ENG USA, vol. 4072, 2000, pages 280-291, XP002352580 ISSN: 0277-786X**

## Description

## INTRODUCTION

The monitoring of heart rate is a typical task in clinical environment for a great variety of patient's condition. The possibility to obtain this information without any contact with the patient represented a future tool in many fields also outside of the clinics.

In fact generally this task cannot be accomplished for when patient's condition determine impairment of their health if contact with skin or any other surface has to be avoided, (e.g. severely burnt subject), or when the type of functional situation e.g. such as the case of people with (e.g. magnetic resonance imaging analysis) or people within confined environment (such as hyperbaric chamber) do not allow to put any sensor or electrodes within the examination environment.

In all these situations an alternative method to the typical one must be provided to monitor heart rate activity at least from the point of view of the beat rate, with respect to heart rate obtained by normally using continuous electrocardiographic records, which the QRS complex detected.

In the following we propose a new technique capable to obtain no contact monitoring of the heart rate using an optical method. One aspect of the invention concerns the application of the laser vibrometry technique to monitoring of physiological quantities (heart rate, respiratory characteristics, simpato-vagal control, etc.) by means of the measurement of the proprieties of the motion of the external surface of human body at several chest windows or directly on several cardiovascular sites such as the carotid one. Laser techniques for vibration measurement, due to their non-contact nature, present an alternative investigational tool with respect to the traditional contact methods (ecg, emg, etc.) to be tested in clinic fields. The invention concerns monitoring a physiological condition of a living thing, and particularly a human, using laser vibrometry to monitor motion of an external surface of the living thing. An advantage of this invention is that it provides a non-contact method to measure the heart rate (HR), and to assess the vital sign monitoring.

The motion of the surface of the external surface of the human body can be related to the mechanical activity of the heart and of the lungs, and is mediated by the tissue motion originated by the quality of the flow and pressure at the heart or at the site of interest together with the compliance of the cardiovascular tree (impedance). In a general sense also the information on the cardiovascular tree condition (e.g. hypertension or compliance affections) could be obtained together with the quality of the beat to beat variability if sufficient long recordings could be made.

Moreover also several functional information could be achieved from the optical signal we carry out from the vibrometer thank to the actual algorithm used to investigate the simpato-vagal control from the neural structure of a patient, that is a mathematical technique capable to obtain the breathing frequency, the impairment of one of nervous system control (simpathethic or vagal nerve) or the incorrect balance among them. In all these cases relevant patient's condition related to the pathology is obtained. Thus Heart rate variability (HRV) can be carried out as one of the most promising markers for the quantitative assessment of autonomic activity of nervous system on heart and its significant consequences on cardiovascular efficiency and post-infarction mortality related to lethal arrhythmias and other cardiovascular pathologies. This is obtained by studying the variation of the interval between consecutive heart beats that is commonly known as heart rate variability (HRV).

The clinical relevance of heart rate variability has been first documented since 1965 (Hon and Lee) in fetal distress. From 1973 (Sayer et al.) research paper have demonstrated the existence of physiological rhythms imbedded in the beat-to-beat heart rate signal. HRV and post-infarction mortality correlation has been firstly demonstrated in 1977 (Wolf) and then widely confirmed since 1980s (see in: Task Force of the European Society of Cardiology and the North American Society of Pacing and Electrophysiology. Heart rate variability: standards of measurement, physiological interpretation, and clinical use, European Heart Journal 1996;17: 354-381).

A review of the alternative methods for non-invasive assessment of cardiac rate suggest the interest on displacement cardiography. Phonocardiology as well as the use of stethoscope can be considered among this class of instruments even if limited to the relative high frequency sensitivity related to audible range of such instruments.

Ballistocardiography has been widely used in the past 40-50 years, it is based on the used of a special low-impedance bed, on which the patient is resting quietly. The bed is sensed by a low-frequency accelerometer which responds to the heart (and body) movements. Special signal processing is needed to allow separation of displacement signals produced by heart from the other interfering inputs. Today the method appears to have not gained the status of a valid clinical diagnostic tool.

Kinetocardiography is using a pressure sensor placed on top of a hollow cylinder, placed on the chest, to detect the pressure waves generated by heart wall displacement. Despite its capability to detect abnormalities in patients with coronary diseases, the technique has not been used widely even because of the low sensitivity of the device.

In apexcardiography, the movement of chest at the point of maximal cardiac impulse is detected and used to detect the presence of coronary artery pathologies. But the low sensitivity of the sensor and the influence of the artefacts on the acquired signal have neglected its diffusion.

In 1990 the seismocardiography has been presented as a novel non invasive technique (although it is a with-contact procedure) for recording and analysing cardiac vibratory activity. The technique uses an ultra low-frequency piezoelectric accelerometer, to be placed on the sternum in the midline to record the compression waves generated by the heart during its cycle and transmitted to the chest wall. The technique demonstrated to be promising but still limited by the contact nature of the transducer and limited to the study of heart rate characteristics.

Power spectral analysis of heart rate variability is a commonly used method in the measurement of sympathovagal interaction on sinus node. Due to the insensitivity of traditional analysis techniques to abrupt, aperiodic changes in heart rate dynamics, dynamic methods (e. g., beat-to-beat analysis techniques) have been developed to uncover stochastic or nonlinear features in heart rate behaviour.

Reciprocal changes in sympathetic and vagal activity have been observed in some physiological conditions, which can be detected by typical changes in spectral components of heart rate variability. In other physiological and pathological states, concomitant sympathetic and vagal activation can occur, leading to accentuated sympathovagal antagonism.

The present research was designed to test the capability to describe both the heart rate behaviour and the sympatho-vagal interaction by generating power spectra and Poincaré plots.

**Final considerations**

The HR measured using the proposed method agreed with the rate derived from an ECG record. This method appears promising as a non-contact method to monitor the cardiac activity of patients under specific conditions, e.g., in incubated newborns, and in severely burnt subjects.

An apparatus and a method according to the preambles of the independent claims is known from document US 2003/0 135 097.

**Demonstration of the Measurement procedure and data processing**

The electrocardiographic (ECG) and the velocity of vibration of the chest wall and the external carotid were simultaneously recorded for 5 minutes. To do this, a two laser head for Doppler vibrometry measurement bench test was built up. After the laying of the two laser beams, ECG and vibrometric signals from the chest wall and the carotid were contemporary recorded and retrieved afterwards to measure the consecutive RR and vibrocardiographic (VV) intervals. Sequences of 415 RR and of the corresponding VV intervals were obtained, on them, tachograms were built. Poincaré maps, that display the relationship between a point and its consecutive in a time series, were obtained. In order to stress the limits of the vibrocardiographic signals in representing the sympatho-vagal activity as the ECG, the power spectral density of the tachograms was performed.

Short-term recordings (5 minutes (Task Force of the European Society of Cardiology and the North American Society of Pacing and Electrophysiology. Heart rate variability: standards of measurement, physiological interpretation, and clinical use, European Heart Journal 1996;17:354-381)) were carried out on healthy human subjects.

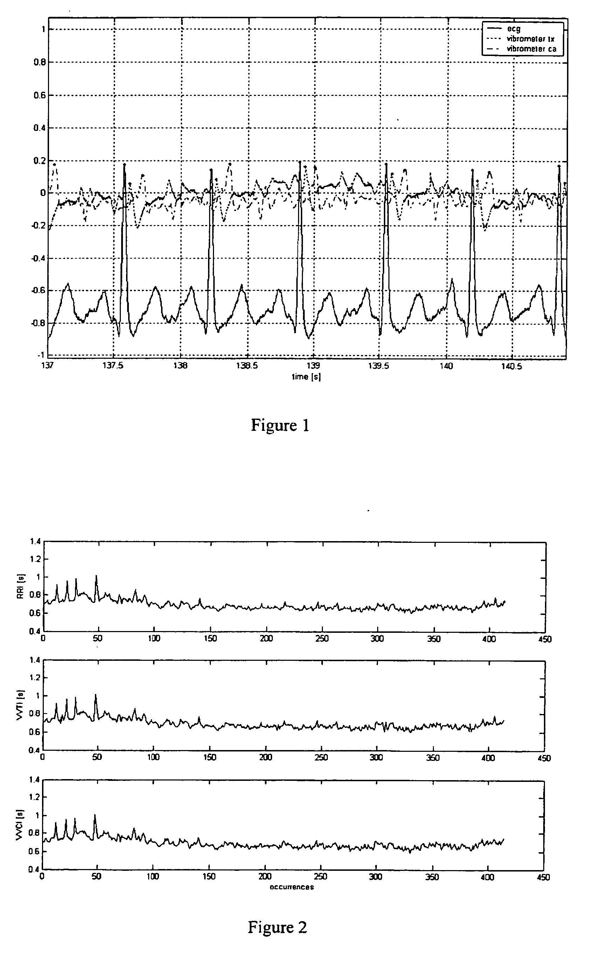

The contemporary recorded ECG and vibrocardiographic signals were not retrieved afterwards to measure the consecutive RR intervals (RRI) and vibrocardiographic intervals both from the thorax (VVIT) and the carotid (VVIC) by using a proper own code for peak detection, by means of MATLAB (The MathWorks, USA) software. An example of peak detection is shown in figure 1. In order to stress the limits of the vibrocardiographic signal in representing the sympatho-vagal activity as the ECG, no atrial or ventricular ectopic beat was deleted (in opposition to the current practice (Kuo et al., 2002)), and for each subject more 5 minutes recordings derived RR intervals and the corresponding VV intervals were obtained. On these RRI and VVIT, VVIC sequences, tachograms were built. For each subject, on the RRI and VVIT, VVIC tachograms a correlation analysis was performed.

Poincaré maps were obtained by plotting $XX_{i+1}$ against $XX_i$ ($XX=RR, VV$, respectively), and the centres of the distributions were calculated.

The measures and the derived quantities for the characterization and quantification of the return maps were performed to assess the relationship between the RRI measures, representative of the heart rate variability (Moraes et al., 2000; Nobrega et al., 2001; Ribeiro et al., 2001; Strumillo 2002; Kuo et al., 2002), and the newly derived quantities from the vibrocardiographic signal.

*Tachograms*

Heart rate variability analysis, shown to be a very powerful tool in the assessment of cardiovascular disease, is the analysis of variations in the instantaneous heart rate time series using beat-to-beat RR intervals, i.e., the RR tachogram (Task Force of the European Society of Cardiology and the North American Society of Pacing and Electrophysiology. Heart rate variability: standards of measurement, physiological interpretation, and clinical use, European Heart Journal 1996;17: 354-381).

The balance between the effects of the two opposite acting branches of the autonomic nervous system (i.e., sympathetic and parasympathetic systems) on heart rate, is referred to as the sympathovagal balance.

It is believed that the beat-to-beat changes of the cardiac cycle (the heart rate may be increased by slow acting sympathetic activity or decreased by fast acting parasympathetic (vagal) activity) is a reflex of the sympathovagal balance (Malik M, Camm AJ. Heart Rate Variability. Armonk, NY: Futura Publishing, 1995).

*Frequency domain analysis*

Power spectral analysis of heart rate variability is a commonly used method in the measurement of sympathovagal interaction on sinus node. Reciprocal changes in sympathetic and vagal activity have been observed in some physiological conditions, which can be detected by typical changes in spectral components of heart rate variability.

The power spectral density analysis was proven to provide the basic knowledge on the frequency content of tachograms. It must be born in mind that the effect of the sympathetic and parasympathetic modulation of the RR intervals in the ECG is typically estimated from spectral analysis of the RR tachogram. The two main frequency bands of interest are referred to as the Low-Frequency (LF) band, $0.04 \div 0.15$ Hz, and the HighFrequency (HF) band, $0.15 \div 0.4$ Hz. Sympathetic tone is believed to influence the LF component whereas both sympathetic and parasympathetic activity have an effect on the HF component (Malik M, Camm AJ. Heart Rate Variability. Armonk, NY: Futura Publishing, 1995; Task Force of the European Society of Cardiology and the North American Society of Pacing and Electrophysiology. Heart rate variability: standards of measurement, physiological interpretation, and clinical use, European Heart Journal 1996;17:354-381). The oscillation in the RR tachogram due to parasympathetic activity, synchronous with the respiratory cycle, is called Respiratory Sinus Arrhythmia. This oscillation manifests itself as a peak in the HF band of the spectrum. For example, as similarly reported in (McSharry PE, Clifford GD, Tarassenko L, Smith LA. A dynamical model for generating synthetic electrocardiogram signals. IEEE Trans Biomed Eng. 2003 Mar; 50(3):289-94), 18 breaths per minute (72 bpm, 1 breath every 4 beats) correspond to a 3.33 seconds oscillation, with a peak in the power spectrum at 0.3 Hz. An additional 10 second oscillation, due to Mayer waves (De Boer RW, Karemaker JM, Strackee J. Hemodynamic fluctuations and baroreflex sensitivity in humans: a beat-to-beat model. Am J Physiol 1987;253:680-689.), often gives rise to a second peak found in the LF band of the power spectrum at approximately 0.1 Hz.

However, only an estimate of the true power spectral density of the tachograms can be obtained, independent of the mathematical methods (properly) employed. In the present study a non parametric method, with the simple, high processing speed, Fast Fourier Transform algorithm, was applied for the calculation of the power spectral density.

*Poincarè plots*

The return map is the plot that displays the relationship between a point and its consecutive point in a time series: its graphic representation is built in mapping a given point in a time series plotted on the x axis, and the next point in the time series plotted on the y axis (Denton et al., 1990).

Linear dynamics is lacking in the complete description of heart rate dynamics. In fact, it was shown that the normal heart rate is not regular, but varies from beat to beat in an irregular manner (Goldberger, 1991; Pool, 1989). Among many tools for the studies of nonlinear dynamics of heart rate, the Poincaré map of RR intervals (RRI) in the ECG signal deserves special attention: in recent years, it has been massively applied to many clinical studies of heart rate variability (Moraes et al., 2000; Nobrega et al., 2001; Ribeiro et al., 2001, Strumillo 2002).

A Poincaré map consists in a scatter-graph built up plotting samples $X_{i+1}$ versus $X_i$. By inspecting the geometric distribution of the map points, information can be obtained about dynamic stability of periodic systems, and presence of deterministic chaos can be put in evidence. Poincaré mapping technique has been shown to be useful for the analysis of dynamic systems displaying periodic/quasi-periodic behaviour It relies on synchonized events, i.e., discrete time observations of the activity of the system under investigation (TS Parker, LO Chua, Practical Numerical Algorithms for chaotic systems. Springler Verlag, New York, 1989). In fact, the Poincaré map is the plot that displays the relationship between a point and its consecutive point in a time series.

As shown in Strumillo and Ruta (2002), the Poincaré mapping technique can be considered as a valid alternative to the generally accepted Fourier spectrum method for detection of dynamics in cardiac repolarization.

It must be born in mind, in the analysis of ECG related Poincaré maps, that they are expected to assume a nonstructured single cluster of points configuration, in normal subjects.

*Subjects*

We have studied 5 healthy adults, without known cardiovascular disease. Informed consent was obtained from subjects. Tests have been conducted during different days in an isolated room, limiting environment noise and potentially influencing emotional circumstances.

**RESULTS**

The results are described in the context of the appended drawings, in which:

Figure 1 shows the recordings on a subject, with the identified peaks both on the ECG and the vibrometric signals from the thorax (VVTI) and the carotid (VVCI);

Figure 2 shows the tachograms built up from the time intervals between consecutive R peaks (RRI), and vibrocardiographic time intervals both from the thorax (VVIT) and the carotid (VVIC);

Figure 3 shows the Poincaré maps for RRI, VVTI and VVCI;

Figure 4 shows the power spectral density (PSD) for the three tacograms; and

Figure 5 shows the beat-by-beat delay times between thoracic and carotid peak.

As results, we report an example of analysis on one subject. Figure 1 shows the recordings on a subject, with the identified peaks both on the ECG and the vibrometric signals from the thorax (VVTI) and the carotid (VVCI).

Figure 2 shows the tachograms built up from the time intervals between consecutive R peaks (RRI), and vibrocardiographic time intervals both from the thorax (VVIT) and the carotid (VVIC) (peaks consecutive in time to the R peak from the ECG)

The correlations between the tacograms were calculated: they are showed in the following:

| Correlation coefficient | RRI vs VVTI | 0.98825 |
| Correlation coefficient | RRI vs VVCI | 0.97675 |
| Correlation coefficient | VVCI vs VVTI | 0.97202 |

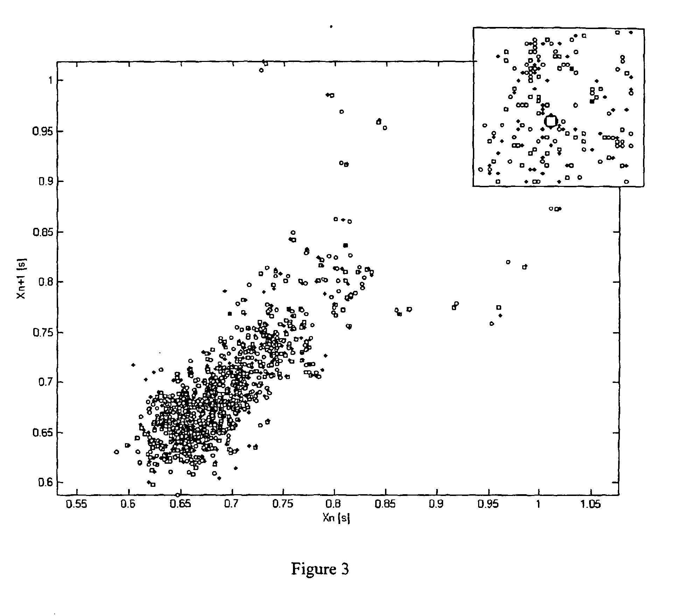

Figure 3 shows the Poincaré maps $X_{n+1}$ vs $X_n$ both for RRI (square "□"), VVTI (solid black diamond "♦") and VVCI (circle "○"). The centres of the distribution were also calculated: it can be noticed that the centres of the three distributions are coincident.

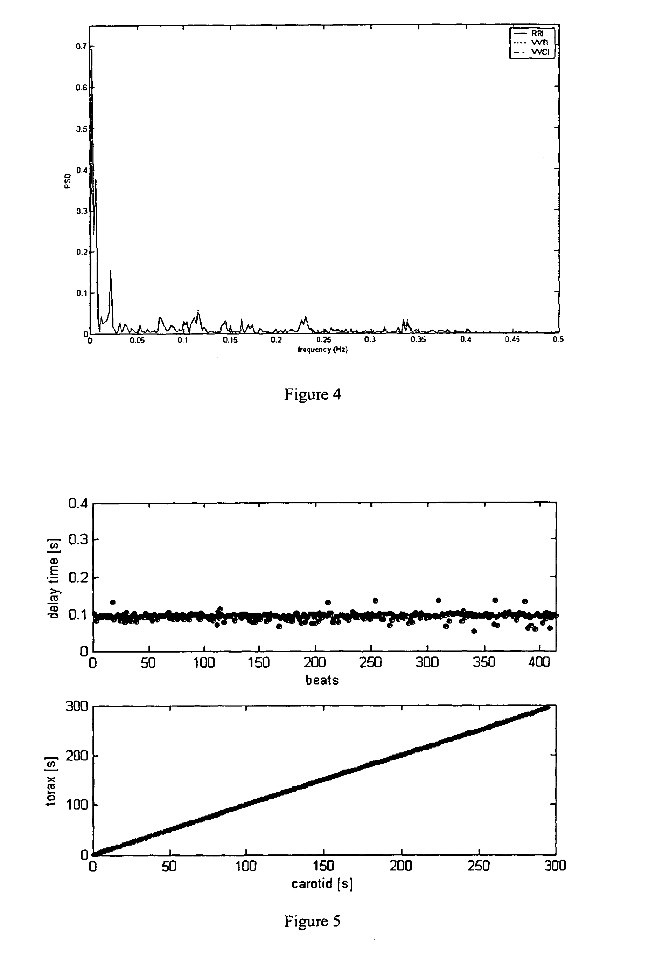

Figure 4 shows the power spectral density (PSD) for the three tacograms, that almost coincident (their mutual correlation coefficients are all 0.999).

Figure 5 shows the beat-by-beat delay times between thoracic and carotid peak. The mean beat-by-beat delay times between thoracic and carotid peak resulted to be $0.0927 \pm 0.0090$s. The linear regression between these beat-by-beat delay times was computer as follows:

$$\text{Time}_{VVTI} = p_1 * \text{Time}_{VVCI} + p_2$$

Coefficients (with 95% confidence bounds):

$P_1 = (1, 1)$
$p_2 = -0.09244 \ (-0.09423, -0.09065)$
$R^2 = 1$

These results clearly shows the linear relationship between the selected events on the vibrometric signals from the thorax and the carotid, respectively.

**Application fields for the technique:**

Some of the potential fields of application for the proposed technique are:

1 - Monitoring of heart rate variability (Cardiology departments, etc.)
2 - Monitoring of heart rate variability and respiratory functions in neonates (Pediatric departments)
3 - Monitoring of patients in remote environments (hyperbaric chambers, decontamination rooms, etc.)
4 - Monitoring of heart rate variability and respiratory functions during magnetic Resonance Imaging.

**Claims**

1. Apparatus for monitoring a physiological condition of a living thing, the apparatus comprising:

   - means for directing a laser beam at a surface of the living thing,
   - means for receiving a reflection of said laser beam incident on said surface, and
   - detecting means operable to detect vibrometric variation in said received reflection with regard to said laser beam, and further operable to determine data indicative of said physiological condition therefrom,
   wherein said physiological condition monitored is the heart rate variability,
   **characterized in that**
   - said means for directing a laser beam comprise two laser head which simultaneously direct the respective laser beams on the chest wall and on the external carotid of the living thing, and
   - said receiving means respectively receive the reflection of the two laser beams incident on the chest wall and on the external carotid.

2. Apparatus according to claim 1, **characterized in that** said means for directing a laser beam are two laser head for Doppler vibrometry measurement.

3. Apparatus according to claim 1 or 2, **characterized in that** said detecting means apply a non parametric method for the calculation of the power spectral density to determine data indicative of said heart rate variability.

4. A method of monitoring a physiological condition of a living thing, the method comprising:

   - directing a laser beam at a surface of said living thing,

- receiving a reflection of said laser beam incident on said surface,
- detecting vibrometric variation in said received reflection with regard to said laser beam, and
- determining, on the basis of said vibrometric variation, data indicative of said physiological condition therefrom,

wherein said physiological condition monitored is the heart rate variability,

**characterized in that**

- said step of directing a laser beam comprises directing simultaneously two laser beams respectively on the chest wall and on the external carotid of the living thing, and
- said step of receiving a reflection comprises receiving the reflections of the two laser beams incident on the chest wall and on the external carotid.

**5.** Method according to claim 4, **characterized in that** said two laser beams are directed by means of two laser head for Doppler vibrometry measurement.

**6.** Method according to claim 4 or 5, **characterized in that** step of determining, on the basis of said vibrometric variation, data indicative of said physiological condition therefrom, comprises of applying non parametric method for the calculation of the power spectral density to determine data indicative of said heart rate variability.

**Patentansprüche**

**1.** Apparat zur Überwachung des physiologischen Zustands eines Lebewesens, Apparat umfassend:

• Vorrichtungen zur Ausrichtung eines Laserbündels auf die Oberfläche eines Lebewesens,
• Vorrichtungen, die dazu geeignet sind, die Reflexion des auf die Oberfläche auftreffenden Laserbündels zu empfangen, und
• Vorrichtungen, die in der Lage sind, die im Produkt der Reflexion des Laserbündels enthaltenen Schwingungen zu erfassen und aus den Schwingungen die Richtwerte des physiologischen Zustands zu bestimmen,
wobei der überwachte physiologische Zustand die Herzvariabilität ist, **dadurch gekennzeichnet, dass**
• die Vorrichtungen zur Ausrichtung des Laserbündels zwei Laserköpfe umfassen, die gleichzeitig die jeweils von ihnen erzeugten Laserbündel auf den Brustkorb im Bereich der Halsschlagader des Lebewesens richten, und
• die Vorrichtungen, die dazu geeignet sind, das Produkt der Reflexion der Laserbündel zu empfangen, jeweils die Reflexion der beiden Laserbündel empfangen, die auf den Brustkorb im Bereich der Halsschlagader auftreffen.

**2.** Apparat nach Anspruch 1, **dadurch gekennzeichnet, dass** die Vorrichtungen für die Erzeugung und den Empfang von Laserbündeln zwei Köpfe von Laser-Doppler-Vibrometern sind.

**3.** Apparat nach Anspruch 1 und 2, **dadurch gekennzeichnet, dass** die Mittel zur Erfassung der Schwingungen ein nicht parametrisches Verfahren für die Berechnung der spektralen Leistungsdichte anwenden, um Richtdaten der Herzvariabilität zu bestimmen.

**4.** Verfahren zur Überwachung des physiologischen Zustands eines lebenden Organismus, umfassend

• Ausrichtung eines Laserbündels auf die Oberfläche des Lebewesens,
• Empfang der Reflexion des auf die Oberfläche auftreffenden Laserbündels,
• Erfassung der vibrometrischen Variationen der Oberfläche unter Bezug auf das Laserbündel, und
• Bestimmung von Richtdaten des physiologischen Zustands auf der Grundlage der vibrometrischen Variation,
wobei der unter Überwachung stehenden physiologisch Zustand die Herzvariabilität ist, **dadurch gekennzeichnet, dass**
• der Schritt der Ausrichtung eines Laserbündels die Verschwenkung zweier Laserköpfe vorsieht, die gleichzeitig die jeweils von ihnen erzeugten Laserbündel auf den Brustkorb im Bereich der Halsschlagader des Lebewesens richten, und
• der zuvor genannte Schritt, der aus dem Empfang des Produkts der Reflexion besteht, den Empfang des Produkts der Reflexion der beiden Laserbündel vorsieht, die auf den Brustkorb im Bereich der Halsschlagader auftreffen.

**5.** Verfahren nach Anspruch 4, **dadurch gekennzeichnet, dass** die beiden Laserbündel unter Verwendung der beiden Köpfe von Laser-Doppler-Vibrometern ausgerichtet werden.

**6.** Verfahren nach Anspruch 4 oder 5, **dadurch gekennzeichnet, dass** Richtdaten des physiologischen Zustands auf der Grundlage der vibrometrischen Variationen bestimmt werden sowie **dadurch**, dass es die Anwendung eines nicht parametrischen Verfahrens für die Berechnung der spektralen Leistungsdichte vorsieht, um Richtdaten der Herzvariabilität zu bestimmen.

**Revendications**

1. Appareil pour la surveillance de l'état physiologique d'un organisme vivant, l'appareil comprenant :

   • des dispositifs aptes à adresser un faisceau laser sur la surface d'un organisme vivant,
   • des dispositifs aptes à recevoir la réflexion du dit faisceau laser, incident sur la dite surface, et
   • des dispositifs en mesure de détecter les vibrations contenues dans le dit produit de la réflexion du dit faisceau laser et de déterminer, en fonction des dites vibrations, les données indicatives du dit état physiologique,
   là où le dit état physiologique surveillé est la variabilité cardiaque, **caractérisé en ce que**
   • les dits dispositifs aptes à adresser le faisceau laser incluent deux têtes laser qui dirigent simultanément les respectifs faisceaux laser qu'elles produisent sur le thorax et en correspondance de l'artère carotide de l'organisme vivant et
   • les dispositifs aptes à traiter le produit de la réflexion des dits faisceaux laser reçoivent respectivement la réflexion des deux faisceaux laser incidents sur le thorax et en correspondance de l'artère carotide.

2. Appareil selon la revendication 1, **caractérisé en ce que** les dispositifs pour la génération et la réception de faisceaux laser sont deux têtes laser pour mesures de vibrométrie laser à effet Doppler.

3. Appareil selon les revendications 1 et 2, **caractérisé en ce que** les dits moyens de détection des vibrations appliquent une méthode non paramétrique pour le calcul de la densité spectrale de puissance aux fins de déterminer des données indicatives de la dite variabilité cardiaque.

4. Une méthode pour la surveillance de l'état physiologique d'un organisme vivant comprenant

   • l'adressage d'un faisceau laser sur la surface du dit organisme vivant,
   • la réception de la réflexion du dit faisceau laser incident sur la dite surface,
   • la détection des variations de la fréquence et de la phase de la dite surface en référence au dit faisceau laser, et
   • la détermination, sur les bases de la dite variation de la fréquence et de la phase, des données indicatives du dit état physiologique,
   là où le dit état physiologique sous surveillance est la variabilité cardiaque, **caractérisés en ce que**
   • la dite opération de diriger un faisceau laser prévoit le pointage de deux têtes laser qui dirigent simultanément les respectifs faisceaux laser qu'elles produisent sur le thorax et en correspondance de l'artère carotide de l'organisme vivant, et
   • la dite opération qui consiste à recevoir le produit de la dite réflexion des deux faisceaux incidents sur le thorax et en correspondance de l'artère carotide.

5. Une méthode, selon la revendication 4, **caractérisée en ce que** les dits deux faisceaux laser sont dirigés en utilisant deux têtes laser pour mesures de vibrométrie laser à effet Doppler.

6. Une méthode, selon les revendications 4 ou 5, **caractérisée en ce que** sont déterminées, sur la base des dites variations de la fréquence et de la phase, des données indicatives de la condition physiologique et qui en fonction de celles-ci comprend l'application d'une méthode non paramétrique pour le calcul de la densité spectrale de puissance aux fins de déterminer des données indicatives de la dite variabilité cardiaque.

Figure 1

Figure 2

8

Figure 3

Figure 4

Figure 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030135097 A

**Non-patent literature cited in the description**

- **Hon ; Lee.** *fetal distress,* 1965
- Heart rate variability: standards of measurement, physiological interpretation, and clinical use. *European Heart Journal,* 1996, vol. 17, 354-381
- **Malik M.** Camm AJ. Heart Rate Variability. Futura Publishing, 1995
- **McSharry PE ; Clifford GD ; Tarassenko L ; Smith LA.** A dynamical model for generating synthetic electrocardiogram signals. *IEEE Trans Biomed Eng,* March 2003, vol. 50 (3), 289-94
- **De Boer RW ; Karemaker JM ; Strackee J.** Hemodynamic fluctuations and baroreflex sensitivity in humans: a beat-to-beat model. *Am J Physiol,* 1987, vol. 253, 680-689
- **TS Parker ; LO Chua.** Practical Numerical Algorithms for chaotic systems. Springler Verlag, 1989